# EUROPEAN PATENT SPECIFICATION

(11) **EP 1 728 786 B1**
(45) Date of publication and mention of the grant of the patent: **13.01.2016**
(21) Application number: 05727095.1
(22) Date of filing: 24.03.2005
(51) Int. Cl.: C07D 261/20

(54) **ONE-POT PROCESS FOR PRODUCING 1,2-BENZISOXAZOLE-3-METHANESULFONAMIDE**
EINTOPFVERFAHREN ZUR HERSTELLUNG VON 1,2-BENZISOXAZOL-3-METHANSULFONAMID
PROCEDE EN UN SEUL POT DE FABRICATION DE 1,2-BENZISOXAZOLE-3-METHANESULFONAMIDE

(30) Priority: 25.03.2004 US 556073 P
(43) Date of publication of application: 06.12.2006
(73) Proprietor: Sumitomo Dainippon Pharma Co., Ltd., Osaka-shi Osaka 541-8524 (JP)
(72) Inventor: UENO, Yoshikazu Dainippon Sumitomo Pharma Co., Ltd, Osaka-shi, Osaka 553-0001 (JP); ISHIKURA, Tsutomu Dainippon Sumitomo Pharma Co,Ltd, Suzuka-shi, Mie 513-0818 (JP)
(74) Representative: Webster, Jeremy Mark
(86) International application number: PCT/JP2005/005349
(87) International publication number: WO 2005/092869

(56) References cited:
- WO-A1-03/031330
- US-A- 3 145 082
- US-A- 3 145 082
- US-A1- 2002 183 525
- US-A1- 2003 114 682
- SHIMIZU M ET AL: "Shinki Kotenkan'yaku Zonisamide no Kaihatsu Kenkyu. /RESEARCH AND DEVELOPMENT OF ZONISAMIDE, A NEW TYPE OF ANTIEPILEPTIC DRUG" YAKUGAKU ZASSHI - JOURNAL OF THE PHARMACEUTICAL SOCIETY OF JAPAN, JAPAN SCIENCE AND TECHNOLOGY INFORMATION AGGREGATOR, ELECTRONIC, JP, vol. 116, no. 7, 1 January 1996 (1996-01-01), pages 533-547, XP002993430 ISSN: 0031-6903
- CASINI, GIOVANNI ET AL: "1,2-Benzisoxazole-3-acetic acid and 3-methyl-1,2-benzisoxazole: a restatement" JOURNAL OF HETEROCYCLIC CHEMISTRY, 6(3), 279 -83 CODEN: JHTCAD; ISSN: 0022-152X, 1969, XP002589446
- SHIMIZU T. ET AL: 'Shinki Kotenkan'yaku Zonisamide no Kaihatsu Kenkyu.' JOURNAL OF THE PHARMACEUTICAL SOCIETY OF JAPAN. vol. 116, no. 7, 1996, pages 533 - 547, XP002993430
- DATABASE CASREACT STN [Online] 1963 MUSTAFA A. ET AL.: 'Subsituted benzopyrano [3,2-c] pyran-2,10-diones and benzopyrano [3,2-c] pyran-2,8-diones.', XP002993281 Database accession no. (60:9720) -& MUSTAFA A.: 'EXPERIMENTS WITH SUBSTITUTED (3,2-c)-PYRANYL-2, 10-DIONES AND BENZOPYRANYL-(3,2-c) PYRAN-2,8-DIONES' TETRAHEDRON vol. 19, no. 11, 1963, pages 1831 - 1839, XP002993431

## Description

### TECHNICAL FIELD

The present invention relates to a process for producing 1,2-benzisoxazole-3-methanesulfonamide being useful as an antiepileptic agent from 4-hydroxycoumarin as a starting compound without isolating intermediates in solid form.

### BACKGROUND ART

Patent Literature 1 discloses a process for producing 1,2-benzisoxazole-3-methanesulfonamide from 1,2-benzisoxazole-3-acetic acid as a starting compound. In this process, the intermediate, sodium 1,2-benzisoxazole-3-methanesulfonate, is isolated in solid form from an aqueous layer, and there is a removal procedure of insoluble materials from a reaction mixture. Processes for producing 1,2-benzisoxazole-3-methanesulfonamide comprising a reaction of isolated sodium 1,2-benzisoxazole-3-methanesulfonate in solid form are disclosed in Patent Literature 2, Patent Literature 3 and Non-Patent Literature 1. Non-Patent Literature 2, Non-Patent Literature 3, Non-Patent Literature 4, Non-Patent Literature 5, Patent Literature 4 and Patent Literature 5 disclose a process for producing 1,2-benzisoxazole-3-acetic acid or its derivatives from 4-hydroxycoumarin or its derivatives. However, these literatures disclose neither a reaction using only water as a solvent nor a reaction involving addition of a chelating agent. Patent Literature 6 and Patent Literature 7 disclose a process for producing sodium 1,2-benzisoxazole-3-methanesulfonate from 1,2-benzisoxazole-3-acetic acid. Patent Literature 8 discloses a process for producing 1,2-benzisoxazole-3-methanesulfonyl chloride from sodium 1,2-benzisoxazole-3-methane-sulfonate, and a process for producing 1,2-benzisoxazole-3-methane-sulfonamide from 1,2-benzisoxazole-3-methanesulfonyl chloride. In Non-Patent Literature 6, an industrial process for producing 1,2-benzisoxazole-3-methanesulfonamide via 1,2-benzisoxazole-3-acetic acid, sodium 1,2-benzisoxazole-3-methanesulfonate and 1,2-benzisoxazole-3-methanesulfonyl chloride as intermediates is disclosed using 4-hydroxy-coumarin as a starting compound. However, these literatures do not disclose a one-pot process for producing 1,2-benzisoxazole-3-methane-sulfonamide using 4-hydroxycoumarin or 1,2-benzisoxazole-3-acetic acid as a starting compound without isolating intermediates in solid form.

Patent Literature 1: JP-A-53-77057
Patent Literature 2: USP 4,172,896
Patent Literature 3: JP-A-54-163823
Patent Literature 4: US 2002/0183525 A1
Patent Literature 5: US 2004/0049053A1
Patent Literature 6: US 2003/0114682 A1
Patent Literature 7: US 2003/0144527 A1
Patent Literature 8: US 2004/0014983 A1
Non-Patent Literature 1: UNO et al., J. Med. Chem., 22, 180 (1979)
Non-Patent Literature 2: A. Mustafa et. al., Tetrahedron, 19, 1831 (1963)
Non-Patent Literature 3: G. Casini, et. al., J. Heterocycl. Chem., 6, 279 (1969)
Non-Patent Literature 4: M. Giannella, et. al., Phytochemistry, 10, 539 (1971)
Non-Patent Literature 5: P. Thourel, et. al., J. Labell. Compd. Radiopharm., 25, 1235 (1988)
Non-Patent Literature 6: Shimizu, et al., Yakugaku-Zasshi, vol. 116, p. 533-547 (1996)

### DISCLOSURE OF INVENTION

### PROBLEMS TO BE SOLVED BY INVENTION

The present inventors have intensively studied an industrially efficient process for producing 1,2-benzisoxazole-3-methanesulfonamide, which is economically excellent and is capable of efficient operations, and have found that when 1,2-dichloroethane is used as a solvent, 1,2-benzisoxazole-3-methanesulfonamide can be produced in one-pot system from 1,2-benzisoxazole-3-acetic acid as a starting compound without isolating intermediates (e.g., sodium 1,2-benzisoxazole-3-methanesulfonate, etc.) in solid form in each step, and when water and 1,2-dichloroethane are used as solvents, 1,2-benzisoxazole-3-methane-sulfonamide can be produced from 4-hydroxycoumarin as a starting compound without isolating intermediates in solid form in each step, and they have accomplished the present invention. Moreover, the present inventors also have intensively studied a process for producing 1,2-benzisoxazole-3-acetic acid, which is a first step of the present process, and have found a process wherein the desired 1,2-benzisoxazole-3-acetic acid can be obtained in high yield by reacting 4-hydroxycoumarin and hydroxylamine using only water as a solvent, and further have found that a rapid decomposition of hydroxylamine, which may unexpectedly occur otherwise, can be suppressed when this reaction is carried out in the presence of a chelating agent, by which the reaction temperature can be easily controlled so that the present process can be made even more desirable.

The present invention provides a process for producing 1,2-benzisoxazole-3-methanesulfonamide, which comprises:
(a) reacting 4-hydroxycoumarin, hydroxylamine or an acid addition salt thereof, and a base in water to give a first mixture;
(b) acidifying the first mixture with an acid, adding 1,2-dichloroethane thereto to give a second mixture, and removing an aqueous layer from the second mixture to give a third mixture containing 1,2-benzisoxazole-3-acetic acid and 1,2-dichloroethane;
(c) removing water from the third mixture by distillation to give a fourth mixture, adding chlorosulfonic acid to the fourth mixture and reacting the mixture to give a fifth mixture, and adding a base to the fifth mixture to give a sixth mixture containing an alkali metal salt of 1,2-benzisoxazole-3-methanesulfonic acid;
(d) adding phosphoryl chloride (also known as phosphorous oxychloride) to the sixth mixture, and reacting the mixture to give a seventh mixture containing 1,2-benzisoxazole-3-methanesulfonyl chloride;
(e) adding ammonia to the seventh mixture and reacting the mixture to give an eighth mixture containing 1,2-benzisoxazole-3-methanesulfonamide; and
(f) isolating 1,2-benzisoxazole-3-methanesulfonamide wherein all of (a) to (e) are conducted without isolating intermediates in solid form.

The reaction in (a) is preferably conducted in the presence of a chelating agent to give the first mixture.

Preferably the first mixture is washed with 1,2-dicholoroethane before acidifying the first mixture in (b).

The present invention also provides a one-pot process for producing 1,2-benzisoxazole-3-methanesulfonamide, which comprises:
(i) reacting 1,2-benzisoxazole-3-acetic acid and chlorosulfonic acid in 1,2-dichloroethane to give a first mixture, and adding a base thereto to give a second mixture containing an alkali metal salt of 1,2-benzisoxazole-3-methanesulfonic acid;
(ii) adding phosphoryl chloride to the second mixture, and reacting the mixture to give a third mixture containing 1,2-benzisoxazole-3-methanesulfonyl chloride;
(iii) adding ammonia to the third mixture and reacting the mixture to give a fourth mixture containing 1,2-benzisoxazole-3-methanesulfonamide; and
(iv) isolating 1,2-benzisoxazole-3-methanesulfonamide, wherein all of (i) to (iii) are conducted in a single reactor without isolating intermediates in solid form.

1,2-benzisoxazole-3-acetic acid may be produced by reacting 4-hydroxycoumarin, hydroxylamine or an acid addition salt thereof, and a base in water, and acidifying the resulting reaction mixture with an acid.

In the above process for producing 1,2-benzisoxazole-3-acetic acid, the reaction of 4-hydroxycoumarin, hydroxylamine or an acid addition salt thereof, and a base is preferably conducted in the presence of a chelating agent.

In the preferred embodiment, the above process for producing 1,2-benzisoxazole-3-acetic acid further comprises washing the reaction mixture of 4-hydroxycoumarin, hydroxylamine or an acid addition salt thereof, and a base with 1,2-dichloroethane before acidifying thereof with an acid.

In further preferred embodiment, the above process for producing 1,2-benzisoxazole-3-acetic acid comprises reacting 4-hydroxycoumarin, hydroxylamine or an acid addition salt thereof, and a base in water in the presence of a chelating agent and washing the resulting reaction mixture with 1,2-dichloroethane before acidifying thereof with an acid.

### MEANS FOR SOLVING THE PROBLEMS

The process for producing 1,2-benzisoxazole-3-methane-sulfonamide of the present invention is a process for producing 1,2-benzisoxazole-3-methanesulfonamide without isolating intermediates in solid form, more particularly, a process for producing 1,2-benzisoxazole-3-methanesulfonamide, which comprises using 4-hydroxy-coumarin as a starting compound, and water and 1,2-dichloroethane as solvents, without isolating intermediates in solid form. Additionally, the process of the present invention may be carried out in a single reactor (one-pot). However, in the above (b), an aqueous layer is removed from the second mixture consisting of two layers, i.e. the aqueous layer and a 1,2-dichloroethane layer. In such liquid-liquid extraction by batch method, a lower layer is usually transferred to another vessel. Therefore, a process for producing 1,2-benzisoxazole-3-methanesulfonamide using multiple vessels, preferably two vessels, is also within the scope of the invention.

The "4-hydroxycoumarin" is commercially available or may be prepared by a conventional method or a modified method thereof.

The "hydroxylamine" may be a commercially available aqueous hydroxylamine solution, but more preferably is prepared by reacting an acid addition salt of hydroxylamine with a base in a reactor.

The "base" includes, for example, sodium hydroxide, potassium hydroxide, sodium carbonate, potassium carbonate, etc. Among them, sodium hydroxide and potassium hydroxide are preferable, and sodium hydroxide is more preferable. Usually, these bases are used in the form of an aqueous solution.

Examples of the "acid addition salt of hydroxylamine" are hydroxylamine hydrochloride, hydroxylamine sulfate, hydroxylamine nitrate, hydroxylamine phosphate, etc., and among them, hydroxylamine sulfate is preferable.

The "chelating agent" includes, for example, ethylenediaminetetraacetic acid, an alkali metal salt of ethylenediaminetetraacetic acid, an ammonium salt of ethylenediaminetetraacetic acid, an alkali metal salt of hydroxyethylethylenediaminetriacetic acid, an alkali metal salt of dihydroxyethylethylenediaminediacetic acid, 1,3-propanediaminetetraacetic acid, diethylenetriaminepentaacetic acid, an alkali metal salt of diethylenetriaminepentaacetic acid, an alkali metal salt of triethylenetetraminehexaacetic acid, an alkali metal salt of hydroxyethyliminodiacetic acid, and hydrates thereof. Preferable chelating agents are ethylenediaminetetraacetic acid, a sodium salt of ethylenediaminetetraacetic acid, a potassium salt of ethylenediaminetetraacetic acid, an ammonium salt of ethylenediaminetetraacetic acid, and hydrates thereof. Among them, a sodium salt of ethylenediaminetetraacetic acid and hydrates thereof are more preferable.

In case that the reaction mixture contains plenty of metal ions (e.g. iron ions), and there is a possibility of a rapid decomposition of hydroxylamine, the reaction temperature can be easily controlled by addition of a chelating agent thereto. The reaction temperature of the present process is usually in the range of 60°C to 100°C, preferably in the range of 80°C to 90°C, more preferably in the range of 84°C to 86°C.

Examples of the "acid" are hydrochloric acid, sulfuric acid, acetic acid, etc., and among them, hydrochloric acid or sulfuric acid is preferable, and sulfuric acid is more preferable.

Since water is contained in the third mixture of 1,2-benzisoxazole-3-acetic acid and 1,2-dichloroethane in (b), the water is removed by distillation from this mixture prior to the reaction with chlorosulfonic acid.

The reaction of 1,2-benzisoxazole-3-acetic acid with chlorosulfonic acid may proceed in the presence or absence of dioxane. The reaction temperature thereof is usually in the range of 60°C to 80°C.

The "alkali metal salt of 1,2-benzisoxazole-3-methanesulfonic acid" is preferably 1,2-benzisoxazole-3-methanesulfonic acid sodium salt.

In the preparation of an alkali metal salt of 1,2-benzisoxazole-3-methanesulfonic acid, an aqueous solution of a base is usually used therein, and hence, water is removed by distillation from a mixture containing an alkali metal salt of 1,2-benzisoxazole-3-methanesulfonic acid prior to the subsequent reaction with phosphoryl chloride.

In the reaction of an alkali metal salt of 1,2-benzisoxazole-3-methanesulfonic acid with phosphoryl chloride, it is preferable to add a tertiary amine such as triethylamine into the reaction mixture. The reaction temperature is usually in the range of 75°C to 85°C.

The "ammonia" is preferably ammonia gas, and the reaction temperature is preferably in the range of 30°C to 60°C.

The isolation step of 1,2-benzisoxazole-3-methanesulfonamide in (f) is carried out by concentrating the reaction mixture, adding water thereto, stirring the mixture, followed by collecting the crystals by filtration. The crystals of 1,2-benzisoxazole-3-methanesulfonamide isolated from the mixed solvent of water and 1,2-dichloroethane are of high purity.

The isolated 1,2-benzisoxazole-3-methanesulfonamide in (f) is further purified by recrystallization to increase the purity thereof. The solvent for recrystallization includes, for example, aqueous ethanol, aqueous isopropanol etc., and among them, aqueous isopropanol is preferable, and isopropanol containing water in an amount of 45 to 55 % by volume is more preferable. By further carrying out azeotropic distillation of 1,2-dichloroethane in the recrystallization step, there are obtained crystals of 1,2-benzisoxazole-3-methanesulfonamide containing residual 1,2-dichloroethane of not more than 5 ppm.

### EFFECTS OF INVENTION

According to the present process, the desired 1,2-benzisoxazole-3-methanesulfonamide can be produced in a one-pot system without isolating intermediates in solid form, and further, the concentration of the residual solvent 1,2-dichloroethane remained therein can be suppressed to 5 ppm or below, and hence, 1,2-benzisoxazole-3-methanesulfonamide, which is useful as a medicament, can be efficiently produced in a quite high purity.

### BEST MODE FOR CARRYING OUT THE INVENTION

The present invention is illustrated in more detail by the following Example, but the present invention should not be construed to be limited thereto. The purity of the products was measured by high performance liquid chromatography.

### Example

1) A mixture of hydroxylamine sulfate (43 g), water (113 ml) and a 25 % aqueous sodium hydroxide solution (57 ml) is stirred, and thereto are added 4-hydroxycoumarin (21 g) and ethylenediaminetetraacetic acid disodium salt dihydrate (0.4 g), and the mixture is stirred with heating at 84°C to 86°C for 4 hours. The reaction mixture is cooled, and thereto are added 1,2-dichloroethane (30 ml) and water (43 ml), and the mixture is stirred. The 1,2-dichloroethane layer is removed, and the pH value of the aqueous layer is adjusted to pH 1-2 with a 62.5 % sulfuric acid. The mixture is extracted twice with 1,2-dichloroethane (120 ml and 10 ml) to give a mixture of 1,2-benzisoxazole-3-acetic acid and 1,2-dichloroethane. The purity of 1,2-benzisoxazole-3-acetic acid in this mixture is 98 %.
2) Water is removed by distillation from the above mixture of 1,2-benzisoxazole-3-acetic acid and 1,2-dichloroethane, and thereto is added dropwise chlorosulfonic acid (15.5 g) while the internal temperature is kept at 63°C-79°C. After the addition, the mixture is stirred for 90 minutes while the reaction temperature is kept at 63°C-79°C. After cooling, a 25 % aqueous sodium hydroxide solution is added to the reaction mixture so as to adjust the pH value thereof to pH 11 or above. Water is removed by distillation from the reaction mixture to give a mixture of sodium 1,2-benzisoxazole-3-methanesulfonate and 1,2-dichloroethane. Triethylamine (2.4 g) and phosphoryl chloride (17.5 g) are added to this mixture, and the mixture is stirred at a temperature of 77°C-83°C for 6 hours. After cooling, to the reaction mixture is added 1,2-dichloroethane (80 ml), and ammonia gas is blown into the mixture until saturated while the reaction temperature is kept at 30°C-60°C. The reaction mixture is concentrated, and water is added thereto. The mixture is stirred, and the precipitated crystals are collected by filtration, and washed with water to give crude crystals of 1,2-benzisoxazole-3-methanesulfonamide. The purity of the crude crystals is 96 %.
3) The above crude crystals are recrystallized from a 50 % aqueous isopropanol, and dried at 80°C for 16 hours to give crystals of 1,2-benzisoxazole-3-methanesulfonamide having a purity of 99 %.

### Synthesis of 1,2-benzisoxazole-3-acetic acid

A mixture of hydroxylamine sulfate (344.0 g), water (904 ml) and a 25 % aqueous sodium hydroxide solution (456 ml) is stirred, and thereto are added 4-hydroxycoumarin (168.0 g) and ethylenediaminetetraacetic acid disodium salt dihydrate (3.2 g), and the mixture is stirred with heating at 84°C-86°C for 4 hours. The reaction mixture is cooled, and thereto is added 1,2-dichloroethane (240 ml). The mixture is stirred, and the aqueous layer is collected. The pH value of the aqueous layer is adjusted to pH 1-2 with a 25 % sulfuric acid, and the precipitated crystals are collected by filtration, washed with water, and dried with air at 60°C for 15 hours to give 1,2-benzisoxazole-3-acetic acid (170.4 g).

### INDUSTRIAL APPLICABILITY

From the above description, the present invention provides a process for producing 1,2-benzisoxazole-3-methanesulfonamide using 1,2-benzisoxazole-3-acetic acid as a starting compound and 1,2-dichloroethane as a solvent, or 4-hydroxycoumarin as a starting compound and water and 1,2-dichloroethane as solvents, by which 1,2-benzisoxazole-3-methanesulfonamide can be effectively prepared in a one-pot system without isolating intermediates in solid form. Moreover, the process may comprise producing 1,2-benzisoxazole-3-acetic acid by reacting 4-hydroxycoumarin and hydroxylamine in water in the presence of a chelating agent.

## Claims

1. A process for producing 1,2-benzisoxazole-3-methane-sulfonamide, which comprises:
(a) reacting 4-hydroxycoumarin, hydroxylamine or an acid addition salt thereof, and a base in water to give a first mixture;
(b) acidifying the first mixture with an acid, adding 1,2-dichloroethane thereto to give a second mixture, and removing an aqueous layer from the second mixture to give a third mixture containing 1,2-benzisoxazole-3-acetic acid and 1,2-dichloroethane;
(c) removing water from the third mixture by distillation to give a fourth mixture, adding chlorosulfonic acid to the fourth mixture and reacting the mixture to give a fifth mixture, and adding a base to the fifth mixture to give a sixth mixture containing an alkali metal salt of 1,2-benzisoxazole-3-methanesulfonic acid;
(d) adding phosphoryl chloride to the sixth mixture, and reacting the mixture to give a seventh mixture containing 1,2-benzisoxazole-3-methanesulfonyl chloride;
(e) adding ammonia to the seventh mixture and reacting the mixture to give an eighth mixture containing 1,2-benzisoxazole-3-methanesulfonamide; and
(f) isolating 1,2-benzisoxazole-3-methanesulfonamide, wherein all of (a) to (e) are conducted without isolating intermediates in solid form.

2. A process for producing 1,2-benzisoxazole-3-methane-sulfonamide according to claim 1, wherein the reaction in (a) is conducted in the presence of a chelating agent to give the first mixture.

3. A process for producing 1,2-benzisoxazole-3-methane-sulfonamide according to claim 2, which further comprises washing the first mixture with 1,2-dichloroethane before acidifying the first mixture in (b).

4. A one-pot process for producing 1 ,2-benzisoxazole-3-methane-sulfonamide, which comprises:
(i) reacting 1,2-benzisoxazole-3-acetic acid and chlorosulfonic acid in 1,2-dichloroethane to give a first mixture, and adding a base thereto to give a second mixture containing an alkali metal salt of 1,2-benzisoxazole-3-methanesulfonic acid;
(ii) adding phosphoryl chloride to the second mixture, and reacting the mixture to give a third mixture containing 1,2-benzisoxazole-3-methanesulfonyl chloride;
(iii) adding ammonia to the third mixture and reacting the mixture to give a fourth mixture containing 1,2-benzisoxazole-3-methanesulfonamide; and
(iv) isolating 1 ,2-benzisoxazole-3-methanesulfonamide, wherein all of (i) to (iii) are conducted in a single reactor without isolating intermediates in solid form.

5. A one-pot process according to claim 4, wherein the base is sodium hydroxide, the alkali metal salt of 1,2-benzisoxazole-3-methanesulfonic acid is sodium 1,2-benzisoxazole-3-methanesulfonate, and ammonia is ammonia gas.

## Patentansprüche

1. Ein Verfahren zur Herstellung von 1,2-Benzisoxazol-3-methansulfonamid, das umfasst:
(a) Umsetzen von 4-Hydroxycumarin, Hydroxylamin oder einem Säureadditionssalz davon und einer Base in Wasser, um ein erstes Gemisch zu erhalten;
(b) Ansäuern des ersten Gemischs mit einer Säure, Hinzufügen von 1,2-Dichlorethan, um ein zweites Gemisch zu erhalten, und Entfernen einer wässrigen Schicht von dem zweiten Gemisch, um ein drittes Gemisch, das 1,2-Benzisoxazol-3-essigsäure und 1,2-Dichlorethan enthält, zu erhalten;
(c) Entfernen von Wasser von dem dritten Gemisch durch Destillation, um ein viertes Gemisch zu erhalten, Zugeben von Chlorsulfonsäure zu dem vierten Gemisch und Umsetzen des Gemischs, um ein fünftes Gemisch zu erhalten, und Zugeben einer Base zu dem fünften Gemisch, um ein sechstes Gemisch, das ein Alkalimetallsalz von 1,2-Benzisoxazol-3-methansulfonsäure enthält, zu erhalten;
(d) Zugeben von Phosphorylchlorid zu dem sechsten Gemisch und Umsetzen des Gemischs, um ein siebtes Gemisch, das 1,2-Benzisoxazol-3-methansulfonylchlorid enthält, zu erhalten;
(e) Zugeben von Ammoniak zu dem siebten Gemisch und Umsetzen des Gemischs, um ein achtes Gemisch, das 1,2-Benzisoxazol-3-methansulfonamid enthält, zu erhalten; und
(f) Isolieren von 1,2-Benzisoxazol-3-methansulfonamid, wobei alle von (a) bis (e) ohne Isolieren von Zwischenprodukten in fester Form durchgeführt werden.

2. Ein Verfahren zur Herstellung von 1,2-Benzisoxazol-3-methansulfonamid gemäß Anspruch 1, wobei die Umsetzung in (a) in der Gegenwart eines Chelatbildners durchgeführt wird, um das erste Gemisch zu erhalten.

3. Ein Verfahren zur Herstellung von 1,2-Benzisoxazol-3-methansulfonamid gemäß Anspruch 2, das ferner Waschen des ersten Gemischs mit 1,2-Dichlorethan vor dem Ansäuern des ersten Gemischs in (b) umfasst.

4. Ein Eintopfverfahren zur Herstellung von 1,2-Benzisoxazol-3-methansulfonamid, das umfasst:
(i) Umsetzen von 1,2-Benzisoxazol-3-essigsäure und Chlorsulfonsäure in 1,2-Dichlorethan, um ein erstes Gemisch zu erhalten, und Hinzufügen einer Base, um ein zweites Gemisch, das ein Alkalimetallsalz von 1,2-Benzisoxazol-3-methansulfonsäure enthält, zu erhalten;
(ii) Zugeben von Phosphorylchlorid zu dem zweiten Gemisch und Umsetzen des Gemischs, um ein drittes Gemisch, das 1,2-Benzisoxazol-3-methansulfonylchlorid enthält, zu erhalten;
(iii) Zugeben von Ammoniak zu dem dritten Gemisch und Umsetzen des Gemischs, um ein viertes Gemisch, das 1,2-Benzisoxazol-3-methansulfonamid enthält, zu erhalten; und
(iv) Isolieren von 1,2-Benzisoxazol-3-methansulfonamid, wobei alle von (i) bis (iii) in einem einzigen Reaktor ohne Isolieren von Zwischenprodukten in fester Form durchgeführt werden.

5. Ein Eintopfverfahren gemäß Anspruch 4, wobei die Base Natriumhydroxid ist, das Alkalimetallsalz von 1,2-Benzisoxazol-3-methansulfonsäure Natrium-1,2-benzisoxazol-3-methansulfonat ist und Ammoniak Ammoniakgas ist.

## Revendications

1. Procédé de production de 1,2-benzisoxazole-3-méthane-sulfonamide, lequel comprend :
(a) la réaction de 4-hydroxycoumarine, d'hydroxylamine ou d'un sel d'addition d'acide de celle-ci, et d'une base dans de l'eau pour fournir un premier mélange ;
(b) l'acidification du premier mélange avec un acide, l'addition de 1,2-dichloroéthane à celui-ci pour fournir un second mélange, et l'élimination d'une couche aqueuse du second mélange pour fournir un troisième mélange contenant de l'acide 1,2-benzisoxazole-3-acétique et du 1,2-dichloroéthane ;
(c) l'élimination d'eau du troisième mélange par distillation pour fournir un quatrième mélange, l'addition d'acide chlorosulfonique au quatrième mélange et la réaction du mélange pour fournir un cinquième mélange, et l'addition d'une base au cinquième mélange pour fournir un sixième mélange contenant un sel de métal alcalin d'acide 1,2-benzisoxazole-3-méthanesulfonique ;
(d) l'addition de chlorure de phosphoryle au sixième mélange, et la réaction du mélange pour fournir un septième mélange contenant du chlorure de 1,2-benzisoxazole-3-méthanesulfonyle ;
(e) l'addition d'ammoniac au septième mélange et la réaction du mélange pour fournir un huitième mélange contenant du 1,2-benzisoxazole-3-méthanesulfonamide ; et
(f) l'isolement de 1,2-benzisoxazole-3-méthanesulfonamide, dans lequel la totalité de (a) à (e) est réalisée sans isoler les intermédiaires dans une forme solide.

2. Procédé de production de 1,2-benzisoxazole-3-méthane-sulfonamide selon la revendication 1, dans lequel la réaction dans (a) est réalisée en présence d'un agent chélatant pour fournir le premier mélange.

3. Procédé de production de 1,2-benzisoxazole-3-méthane-sulfonamide selon la revendication 2, lequel comprend de plus le lavage du premier mélange avec du 1,2-dichloroéthane avant l'acidification du premier mélange dans (b).

4. Procédé à un seul réacteur pour produire du 1,2-benzisoxazole-3-méthanesulfonamide, lequel comprend :
(i) la réaction d'acide 1,2-benzisoxazole-3-acétique et d'acide chlorosulfonique dans du 1,2-dichloroéthane pour fournir un premier mélange, et l'addition d'une base à celui-ci pour fournir un second mélange contenant un sel de métal alcalin d'acide 1,2-benzisoxazole-3-méthanesulfonique ;
(ii) l'addition de chlorure de phosphoryle au second mélange, et la réaction du mélange pour fournir un troisième mélange contenant du chlorure de 1,2-benzisoxazole-3-méthanesulfonyle ;
(iii) l'addition d'ammoniac au troisième mélange et la réaction du mélange pour fournir un quatrième mélange contenant du 1,2-benzisoxazole-3-méthanesulfonamide ; et
(iv) l'isolement de 1,2-benzisoxazole-3-méthanesulfonamide, dans lequel la totalité de (i) à (iii) est réalisée dans un réacteur unique sans isoler d'intermédiaires dans une forme solide.

5. Procédé à un seul réacteur selon la revendication 4, dans lequel la base est l'hydroxyde de sodium, le sel de métal alcalin d'acide 1,2-benzisoxazole-3-méthanesulfonique est le 1,2-benzisoxazole-3-méthanesulfonate de sodium, et l'ammoniac est du gaz d'ammoniac.
